# EUROPÄISCHE PATENTSCHRIFT

(11) **EP 1 276 889 B1**
(45) Veröffentlichungstag und Bekanntmachung des Hinweises auf die Patenterteilung: **19.10.2005**
(21) Anmeldenummer: 01942975.2
(22) Anmeldetag: 27.04.2001
(51) Int. Cl.: C12N 15/90, C12N 15/67, C12N 5/16, A01K 67/027

(54) **SLEEPING BEAUTY, EIN TRANSPOSONVEKTOR MIT BREITEM WIRTSBEREICH FÜR DIE GENETISCHE TRANSFORMATION BEI WIRBELTIEREN**
SLEEPING BEAUTY, A TRANSPOSON VECTOR WITH A BROAD HOST RANGE FOR THE GENETIC TRANSFORMATION IN VERTEBRATES
SLEEPING BEAUTY, UN VECTEUR TRANSPOSON A LARGE GAMME D'HOTES POUR LA TRANSFORMATION GENETIQUE CHEZ LES VERTEBRES

(30) Priorität: 27.04.2000 DE 10020553
(43) Veröffentlichungstag der Anmeldung: 22.01.2003
(73) Patentinhaber: MAX-DELBRÜCK-CENTRUM FÜR MOLEKULARE MEDIZIN, 13125 Berlin (DE)
(72) Erfinder: IVICS, Zoltan, 13125 Berlin (DE); IZSVAK, Zsuzsanna, 13125 Berlin (DE); Zayed, Hatem, 13359 Berlin (DE)
(74) Vertreter: Graf von Stosch, Andreas
(86) Internationale Anmeldenummer: PCT/DE2001/001595
(87) Internationale Veröffentlichungsnummer: WO 2001/081565

(56) Entgegenhaltungen:
- WO-A-98/40510
- WO-A-99/25817

## Beschreibung

### STAND DER TECHNIK

Für die Entwicklung von In-vivo-Genübertragungsstrategien zur Behandlung ererbter und erworbener Erkrankungen beim Menschen (somatischer Gentransfer) sowie zur Transgenese bestimmter Wirbeltierarten für die landwirtschaftliche und medizinische Biotechnologie wurden erhebliche Anstrengungen unternommen. Für eine wirksame Gentherapie ist folgendes nötig:
1) Erreichung der Übertragung therapeutischer Gene mit hoher Effizienz, insbesondere in relevante Zellen,
2) Expression des Gens über einen längeren Zeitraum,
3) Sicherstellung der Unschädlichkeit beim Einbringen des therapeutischen Gens.

Für die Genübertragung zum Zweck der Gentherapie beim Menschen werden mehrere Methoden und Vektoren eingesetzt (Verma und Somia, 1997). Diese Methoden lassen sich grob in virale und nicht virale Technologien unterteilen, und alle haben Vorteile und Grenzen; keine von ihnen bietet eine perfekte Lösung. Im allgemeinen besitzen Vektoren, die in der Lage sind, das Transgen zu integrieren, auch die Fähigkeit, eine längere Expression zu sichern. Andererseits ist die zufällige Integration in Chromosomen wegen der möglichen Unterbrechung der endogenen Genfunktion an und in der Nähe der Insertionsstelle ein Problem.

Die Anpassung von Viren für den Gentransfer ist eine verbreitete Methode, die genetische Konstruktion des Vektors ist aber aufgrund der Beschränkungen des Virus hinsichtlich Größe, Struktur und Expressionsregulation begrenzt. ***Retrovirale Vektoren*** (Miller, 1997) integrieren wirksam fremde DNA in die Chromosomen transduzierter Zellen und besitzen ein enormes Potential zur lebenslangen Genexpression. Der Zeitaufwand und die Finanzmittel, die für ihre Präparation erforderlich sind, können für die großtechnische Produktion jedoch ungeeignet sein. Darüber hinaus sind mehrere weitere Faktoren zu berücksichtigen, darunter die Sicherheit, die zufällige Chromosomenintegration und die Notwendigkeit der Zellreplikation für die Integration. *Lentivirale Systeme,* die auf dem humanen Immundefektvirus (HIV) beruhen, gehören zu den Retroviren, aber sie können teilende und nicht teilende Zellen infizieren. ***Adenovirale Vektoren*** sind nachweislich zur In-vivo-Genübertragung von Transgenen für eine breite Vielzahl teilender und nicht teilender Zellen sowie zur Vermittlung einer hohen, jedoch kurzzeitigen Genexpression in der Lage. Adenoviren besitzen nicht die Fähigkeit, das transferierte Gen in chromosomale DNA zu übertragen, und ihre Präsenz in Zellen ist kurzlebig. So müssen rekombinante Adenovirenvektoren wiederholt verabreicht werden und erzeugen aufgrund der Immunogenität des Vektors eine unerwünschte Immunreaktion bei Menschen. ***Adeno-assoziierte Virus*** (AAV)-Vektoren besitzen mehrere mögliche Vorteile, die zu untersuchen sind, darunter die Möglichkeit der gezielten Integration des Transgens. Eine der deutlichsten Beschränkungen des AAV-Vehikels ist die geringe maximale Insertionsgröße (3,5-4,0 kb). Zurzeit wurden (hybride) Kombinationsvektoren (retroviral/adenoviral, retroviral/AAV) entwickelt, die bestimmte Probleme der einzelnen Viralvektorsysteme lösen können.

Nicht virale Methoden, darunter DNA-Kondensationsmittel, *Liposome, Mikroinjektion* und "Genpistolen" könnten einfacher und sicherer in der Anwendung sein als Viren. Die Eintritts- und Aufnahmeeffizienz der bloßen DNA ist jedoch gering. Diese kann durch Verwendung von Liposomen gesteigert werden. Im Allgemeinen sind die gegenwärtig verwendeten nicht viralen Systeme nicht geeignet, die Integration in Chromosomen zu fördern. Folglich sind stabile Gentransferfrequenzen unter Verwendung nicht viraler Systeme sehr niedrig. Außerdem führen die meisten nicht viralen Methoden häufig zu Konkatamerisation sowie zu zufälligen Brüchen in der Eingabe-DNA und diese wiederum u.U. zur Genstillegung.

Zurzeit existierne keine oder wenige Genübertragungssysteme bei Wirbeltieren für somatischen Gentransfer, die folgende Eigenschaften verbinden:
1) Fähigkeit zur Übertragung von Genen in vivo;
2) breiter Wirts- und Gewebebereich;
3) stabile Geninsertion in Chromosomen;
3) zuverlässige, langzeitige Expression der übertragenen Gene;
4) Sicherheit;
5) kostengünstige großtechnische Produktion.

### BESCHREIBUNG

Transponierbare Elemente oder kurz Transposons sind mobile Segmente der DNA, die sich innerhalb von Genomen von einem Ort zu einem anderen bewegen können (Plasterk u. a., 1999). Diese Elemente bewegen sich über einen konservativen "Ausschneiden-und-Einfügen"-Mechanismus: die Transposase katalysiert die Exzision des Transposons aus seiner ursprünglichen Position und fördert seine Reintegration an einer anderen Stelle im Genom. Transposase-defiziente Elemente können mobilisiert werden, wenn durch ein anderes Transposasegen die Transposase *in trans* bereitgestellt wird. So können Transposons als Vehikel genutzt werden, um neue Phänotypen durch Transgenese in Genome einzubringen. Sie sind nicht infektiös, und da sie an ihren Wirt angepasst werden müssen, hielt man sie für weniger schädlich für den Wirt als Viren.

DNA-Transposons werden routinemäßig für Insertionsmutagenese, Genmapping und Gentransfer in feststehenden Modellsystemen, die keine Wirbeltiere umfassen, wie z.B. *Drosophila melanogaster oder Caenorhabditis elegans*, sowie bei Pflanzen verwendet. Transponierbare Elemente wurden jedoch aus zwei Gründen nicht für die Untersuchung von Wirbeltiergenomen verwendet. Erstens gab es bei diesen Arten bisher keine genau definierten mobilen Elemente auf DNA-Basis. Zweitens war bei Tieren die Artspezifität der Transposition aufgrund der Notwendigkeit von Faktoren, die durch den natürlichen Wirt erzeugt wurden, ein Haupthindernis für den Transfer eines aktiven Transposonsystems von einer Art auf eine andere.

*Sleeping Beauty* (SB) ist ein aktives, Tc1-ähnliches Transposon, das aus Stücken und Teilen inaktiver Elemente rekonstruiert wurde, die in Genomen des Teleostfisches zu finden sind. SB ist derzeit das einzige aktive Transposonsystem auf DNA-Basis wirbeltierischen Ursprungs, das im Labor unter Verwendung molekularbiologischer Standardtechniken manipuliert werden kann (WO 98/40510 und WO 99/25817). SB bewirkt in Zellen von Fischen, Mäusen und Menschen eine effiziente und präzise Transposition durch Ausschneiden und Einfügen (Ivics u. a., 1997; Luo u. a., 1998; Izsvak et al.,2000; Yant et al., 2000).

Einige Hauptmerkmale eines wünschenswerten Transposonvektors sind: einfache Verwendung, relativ breiter Wirtsbereich, geringe Größen- oder Sequenzbeschränkungen, effiziente Chromosomenintegration und stabile Erhaltung einer zuverlässigen Transgenexpression über mehrere Generationen transgener Zellen und Organismen. *Sleeping Beauty* erfüllt diese Anforderungen auf der Grundlage der folgenden Ergebnisse.

### VERSUCHSERGEBNISSE

***Sleeping Beauty*** **ist in verschiedenen Wirbeltierarten aktiv.** Um die Beschränkungen der Wirtsspezifität von SB bei Wirbeltieren zu bewerten, wurden kultivierte Zellen von Vertretern verschiedener Wirbeltierklassen unserer standardisierten Transpositionsanalyse unterzogen. Zelllinien von sieben verschiedenen Fischarten, drei von der Maus, zwei vom Menschen und je eine von einem Frosch, einer Wachtel, einem Schaf, einer Kuh, einem Hund, einem Kaninchen, einem Hamster und einem Affen wurden untersucht. Wie in Tabelle 1 zusammengefasst, war SB in der Lage, die Frequenz der Transgenintegration in all diesen Zelllinien mit Ausnahme der Wachtel zu erhöhen. Wir sind deshalb zu dem Schluss gekommen, dass SB im wesentlichen bei allen Wirbeltierarten wirksam wäre (Izsvak et al., 2000).

### Auswirkungen der Transposongröße auf die Effizienz der Transposition durch

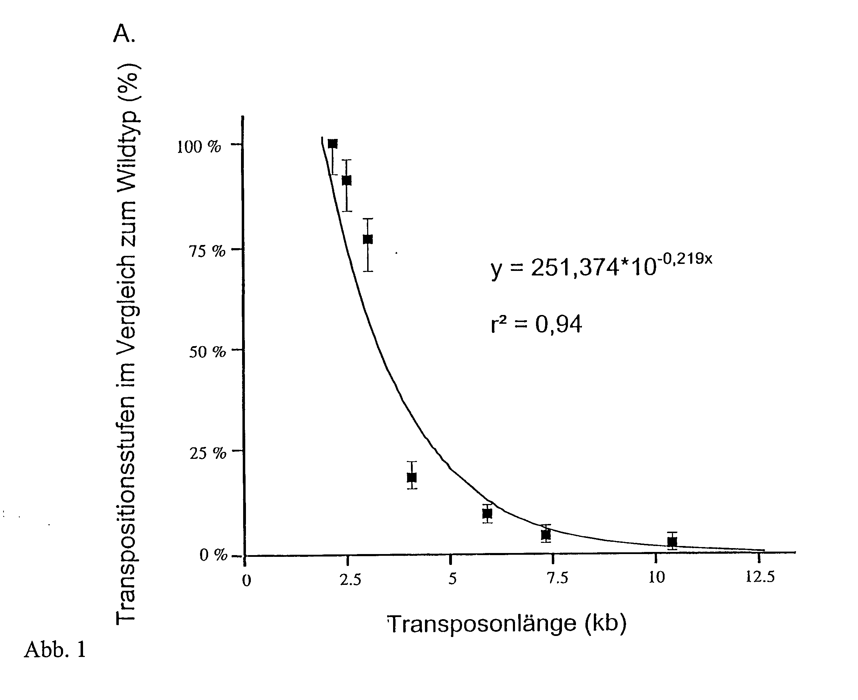

***Sleeping Beauty.*** Die natürliche Größe von SB beträgt ca. 1,6 kb. Um als Vektor für somatische und Keimstammtransformation verwendbar zu sein, muss ein Transposonvektor in der Lage sein, große DNA-Fragmente (mehrere kb), die komplette Gene enthalten, einzubringen und die Fähigkeit bewahren, durch eine Transposase wirksam mobilisiert zu werden. Um die Größenbeschränkungen des SB-Systems zu bestimmen, wurden eine Reihe von Spenderkonstrukten getestet, die Transposons zunehmender Länge enthielten (2,2; 2,5; 3,0; 4,0; 5,8; 7,3 und 10,3 kb). Ähnlich wie bei anderen Transposonsystemen transponierten größere Elemente weniger effizient, und bei jeder kb-Erhöhung der Transposonlänge stellten wir eine exponentielle Abnahme der Transpositionseffizienz von etwa 30 % fest (Abb. 1) (Izsvak et al, 2000). Es wurde festgestellt, dass die Maximalgröße von SB-Vektoren ähnlich wie bei den meisten retroviralen Vektoren ca. 10 kb beträgt. Obgleich die Transpositonseffizienz im allgemeinen mit wachsender Vektorgröße abzunehmen scheint, ist die Obergrenze offensichtlich nicht so streng wie bei retroviralen Vektoren. Außerdem erhöht eine Reduzierung der DNA-Länge außerhalb des Transposons im Normalfall die Effizienz der Transposition (- 30 % Zunahme/kb) (Izsvak et al., 2000). Mit anderen Worten: bei einer bestimmten Insertionsgröße kann die Transpositionseffizienz gesteigert werden, indem die beiden invertierten Repeats des Transposons auf einem kreisförmigen Plasmidmolekühl angenähert werden.

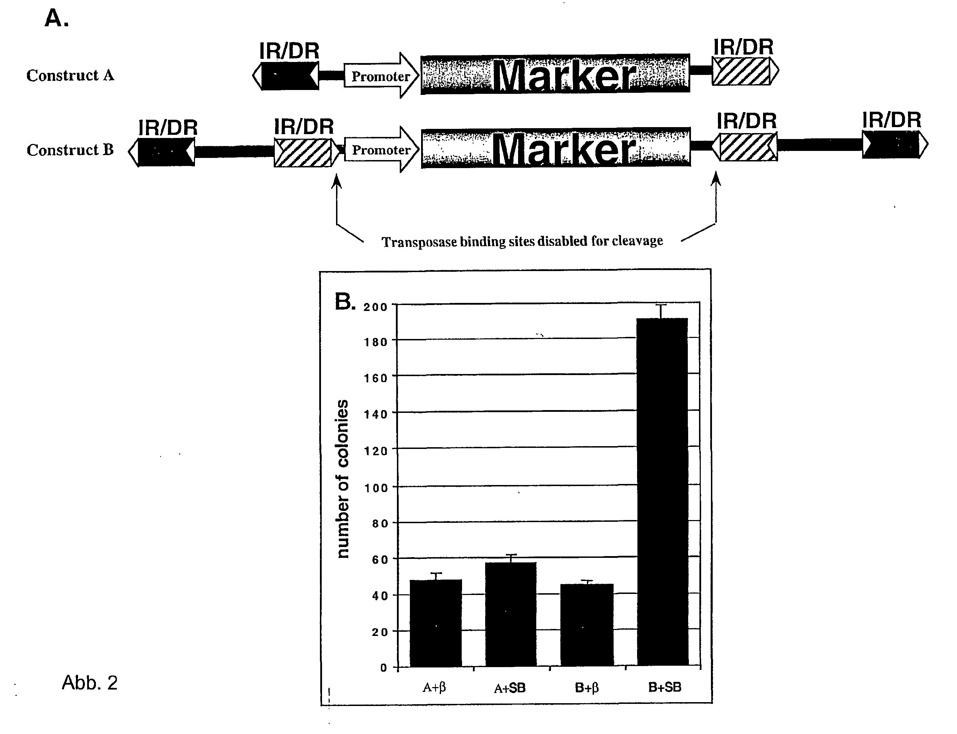

Ein 14 kb- DNA-Stück, flankiert von einem Paar *Pariser* Elemente, scheint nach *Drosophila virilis* transponiert worden zu sein.Diese Art von "Sandwich"-Anordnungen zweier kompletter SB-Elemente, die ein Transgen flankieren, erhöht die Fähigkeit des Vektors, größere DNA-Stücke zu transponieren. So wurde ein etwa 5 kb-DNA-Stück mit zwei intakten SB-Kopien in umgekehrter Richtung flankiert (Abb. 2A). Der Vektor war so beschaffen, dass die Transposase in der Lage war, an ihre inneren Bindestellen in jedem Element zu binden, ihre Fähigkeit, DNA an diesen Stellen abzuspalten, jedoch einbüßte. Die Transpositionsleistung des Sandwichelementes wurde um etwa das 4-fache erhöht, verglichen mit einem SB-Vektor, der das gleiche Markergen enthielt (Abb. 2B). So kann der Sandwich-Transposon-Vektor für die Erweiterung der Klonungsfähigkeit von SB-Elementen für die Übertragung großer Gene, deren stabile Integration in Genome mit den derzeitigen viralen und nichtviralen Vektoren problematisch war, vorteilhaft eingesetzt werden. Die vorliegende Anmeldung bezieht sich auf diesen sogennanten Sandwich-Transposor-Vektor, wie zum Beispiel in der Abbildung 2A, konstrukt B, dargestellt.

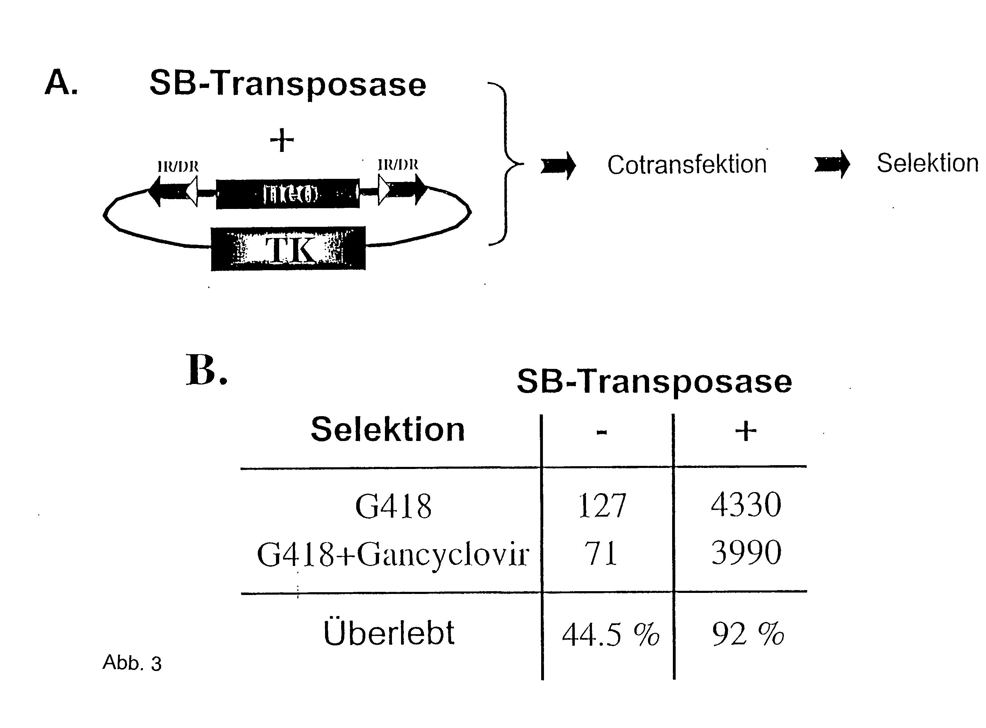

***Sleeping Beauty*** **integriert präzise.** Unsere Analyse einer Handvoll zufällig ausgewählter SB-Insertionsstellen in HeLa-Zellen hat ergeben, dass die Chromosomenintegration in allen Fällen präzise und von einer Duplizierung der TA-Zieldinukleotide (Ivics u. a., 1997), einer molekularen Signatur der *Tcl*/*mariner*-Transposition, begleitet war. Um das Verhältnis der präzisen gegenüber den nicht präzisen Integrationsereignissen in einem größeren Maßstab zu bestimmen, wurde ein genetischer Assay für die Positiv-Negativ-Selektion entwickelt. Dieser Assay wählt positiv nach der Integration von Transposonsequenzen (präzise Ereignisse) und negativ anhand von Zellen aus, die integrierte Vektorsequenzen in ihren Chromosomen tragen (nicht präzise Ereignisse). Das Thymidinkinase-(TK)-Gen des Herpes-simplex-Virus Typ 1 wurde in das Vektorgerüst von pT/neo eingebaut. Bei einer Cotransfektion dieses Konstrukts in Zellen zusammen mit einem SB-Transposase-Expressionsplasmid wurden G-418-resistente Kolonien in Gegenwart oder in Abwesenheit von Gancyclovir selektiert, das toxisch für Zellen ist, in denen das TK-Gen exprimiert wird. Etwa 90 % der G-418-resistenten HeLa-Kolonien überlebten die Gancyclovir-Selektion, was zeigt, dass die Mehrzahl der Integrationsereignisse das toxische TK-Gen nicht einschloss; dies ist ein Maß der präzisen, transposase-vermittelten Integrationsereignisse (Abb. 3). Ähnliche Ergebnisse, die die präzise Transposition zeigen, wurden bei Hamster-K1-, Elritzen-FHM- und Maus-3T3-Zellen erzielt (Izsvak et al. 2000). Unsere Ergebnisse zeigen eine hohe Genauigkeit der Substraterkennung und präzise SB-Transposition selbst bei diesen phylogenetisch entfernten Zellstämmen. Das SB-System sorgt für präzise Integration des gewünschten Gens, nur flankiert von den kurzen, invertierten Wiederholungssequenzen (230 bp). Diese Genauigkeit der Integration bedeutet, dass Plasmidsequenzen, die antibiotische Resistenzgene tragen, zurückgelassen werden und sich nicht in das Wirtsgenom integrieren; damit wird ein allgemeines Problem der Gentherapie und Transgenese gelöst.

Im Gegensatz zur Konkatamerisation extrachromosomaler DNA, die häufig bei Verwendung nicht viraler Gentransfermethoden angetroffen wird, integrieren sich SB-Transposons als einzelne Kopien.

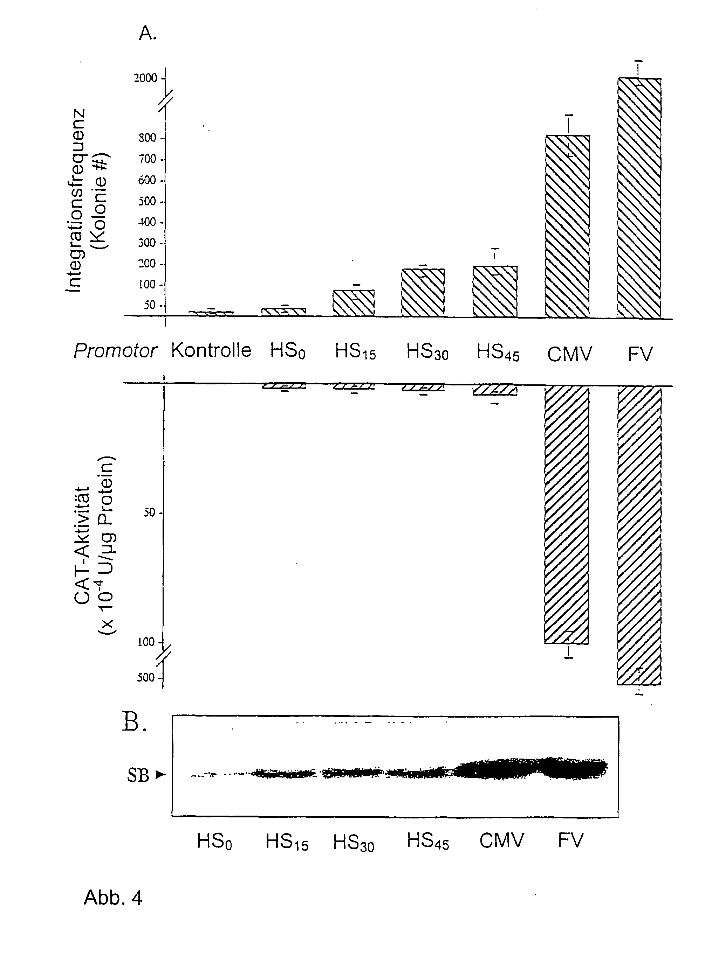

**Die SB-Expression kann durch viele unterschiedliche Promotoren erfolgen, um die Transposaseexpression für eine Vielzahl von Anwendungen zu optimieren.** Für die Expression der SB-Transposase wurden drei verschiedene Promotoren verwendet, und zwar des humanen Hitzeschock-70-Gens (HS), des frühen humanen Cytomegalovirus-Gens (CMV) und des Carp-β-Actin-Gens (FV). HS ist durch Anwendung eines Hitzeschocks auf transfizierte Zellen induzierbar, während CMV und FV als "starke" konstitutive Promotoren betrachtet werden können. Wie in der oberen Graphik von Abbildung 4 gezeigt, nahmen die Zahlen der G-418-resistenten Zellen unter Verwendung von HS-SB und durch Verlängerung der Induktionszeit (15 min, 30 min und 45 min) ebenfalls zu. Die durch den CMV-Promotor aktivierte Transposase erzeugte eine erheblich höhere Zahl von Kolonien, und wir erhielten noch größere Zahlen mit FV-SB (Izsvak et al., 2000). Wir haben die relativen Stärken der drei Promotoren in der Genexpression durch Messung der Reporterenzymaktivität von Chloramphenicolacetyltransferase (CAT) anhand vorübergehend transfizierter Zellen bewertet. Die Höhen der CAT-Aktivität bei Expression durch dieselben Promotoren unter denselben Versuchsbedingungen zeigten etwa die gleichen Verhältnisse wie wir sie für Transpositionsaktivitäten erzielt haben (Abb. 4, untere Graphik).

Wir kamen zu dem Schluss, dass die Zahl der Transpositionsereignisse pro transfizierter Zellpopulation direkt proportional der Zahl der in den Zellen vorhandenen Transposasemoleküle ist. So hat Überexpression von Transposase offensichtlich keine hemmende Wirkung auf die SB-Transposition, zumindest nicht in dem Expressionsbereich, in dem SB bei den meisten transgenen Experimenten verwendet würde, und somit ist die SB-Expression durch viele unterschiedliche Promotoren möglich, um die Transposaseexpression für eine Vielzahl von Anwendungen zu optimieren.

**Das Transposon** ***Sleeping Beauty*** **vermittelt die Insertion fremder Gene in die Genome von Wirbeltieren in vivo.** Im Gegensatz zu viralen Vektoren können ungeheure Mengen von Plasmidvektoren problemlos zu sehr geringen Kosten erzeugt, gereinigt und unterhalten werden. *Sleeping Beauty* ist das erste nicht virale System, das die plasmidcodierte Genintegration und Langzeitexpression in vivo ermöglicht.

Unter Verwendung der Schwanzvenen-Injektionstechnik bloßer DNA hat die Transposase Sleeping Beauty wirksam die Transposonintegration in mehrere nicht codierende Regionen des Mausgenoms in vivo bewirkt. Die DNA-Transposition tritt in etwa 5-6 Prozent der transfizierten Mauszellen auf und führt zu Langzeitexpression (> 3 Monate) therapeutischer Konzentrationen des humanen Gerinnungsfaktors IX in vivo (Yant et al., 2000). Diese Resultate stellen die DNA-vermittelte Transposition als leistungsfähiges neues genetisches Werkzeug für Wirbeltiere unter Beweis und bieten faszinierende neue Strategien zur Verbesserung bestehender nicht viraler und viraler Vektoren für Transgenese und gentherapeutische Anwendungen beim Menschen.

**Die invertierten Repeatsequenzen von** ***Sleeping Beauty*** **tragen keine Promotor- und/oder Verstärkerelemente, die potentiell benachbarte Genexpression bei Integration in das Genom beeinflussen können.** Um zu prüfen, ob die invertierte Wiederholungssequenz des Transposons Sleeping Beauty Promotorelemente trägt, wurde der folgende Versuch durchgeführt. Das lacZ-Gen wurde im Rahmen des SB-Transposasegens in ein Konstrukt eingebaut, das die invertierten Transposon-Repeatsequenzen stromaufwärts der Expressionseinheit beibehielt. Mit diesem Konstrukt transfizierte humane HeLa-Zellen wurden entweder *in situ* gefärbt, oder Zellextrakte wurden in einer *In-vivo*-Analyse auf β-Galactosidaseaktivität untersucht. In beiden Fällen wurde keine nachweisbare β-Galactosidaseaktivität erzielt. Dies deutet darauf hin, dass den invertierten Wiederholungen keine signifikante Promotoraktivität übertragen werden konnte.

Zur Untersuchung der Verstärkeraktivität wurde die linke invertierte Wiederholung des SB-Transposons mit einem minimalen TK-Promotor vor dem Luciferase-Markergen verschmolzen. Der humane Cytomegalovirus-Verstärker (CMV-Verstärker) diente als positive Kontrolle. An der invertierten Wiederholungssequenz von *Sleeping Beauty* wurde keine signifikante Verstärkeraktivität beobachtet. Im Gegensatz zu Retroviren, deren LTRs Verstärker-/Promotorelemente enthalten, sind SB-Vektoren in der Transkription neutral und würden somit Muster endogener Genexpression nicht verändern.

**Einzelne Aminosäuresubstitutionen an nicht wesentlichen Stellen im Transposase-Polypeptid ändern die Transposaseaktivität nicht.** Eukaryotische Expressionsplasmide sind alle Derivate des pCMV/SB-Konstruktes, der bereits beschrieben wurde (Ivics et al., 1997). **pCMV/SB-S116V** wurde durch PCR-Amplifikation von pCMV/SB mit Primern 5'-CCGCGTCGCGAGGAAGAAGCCACTGCTCCAA-3' und 5’-CTTCCTCGCGACGCGGCCTTTCAGGTTATGTCG-3’, wobei das PCR-Produkt mit dem Restriktionsenzym *Nru*I, dessen Erkennungsequenz in den Primersequenzen unterstrichen ist, abgespalten und mit T4 DNA-Ligase rezirkuliert wird. Die Mutationssequenz mit den kodierten Aminosäuren ist wie folgt:

109 110 111 112 113 114 115 116 117 118 119 120 121 122 123 124
CGA CAT AAC CTG AAA GGC CGC GTC GCG AGG AAG AAG CCA CTG CTC CAA
R H N L K G R V A R K K P L L Q

Die Mutation einer einzigen Aminosäure verändert sich in Position 116, die jetzt ein Valin (fett gedruckt) anstelle des ursprünglichen Serin ist.

**pCMV/SB-N280H** wurde durch PCR-Amplifkation von pCMV/SB mit Primern, 5' -GCCCAGATCTCAATCCTATAGAACATTTGTGGGCAGAACTG- 3' und 5' -ATTGAGATCTGGGCTTTGTGATGGCCACTCC- 3', wobei das PCR-Produkt mit dem Restriktionsenzym *Bg*/*II*, dessen Erkennungsequenz in den Primersequenzen unterstrichen ist, abgespalten und mit T4 DNA-Ligase rezirkuliert wird. Ein Teil der Mutationssequenz mit den kodierten Aminosäuren ist wie folgt:

270 271 272 273 274 275 276 277 278 279 280 281 282 283 284 285
TCA CAA AGC CCA GAT CTC AAT CCT ATA GAA CAT TTG TGG GCA GAA CTG
S Q S P D L N P I E H L W A E L

Die Mutation ist eine einzige Aminosäureveränderung in Position 280, die jetzt ein Histidin (fett gedruckt) anstelle des ursprünglichen Asparagins ist.

**pCMV/SB-S58P** wurde durch die PCR-Amplifikation eines DNA-Fragments an der Verbindung des CMV-Promoters und des Transposasegens in pCMV/SB mit Primern 5'-GGTGGTGCAAATCAAAGAACTGCTCC-3' und 5’-CAGAACGCGTCTCCTTCCTGGGCGGTATGACGGC-3’, und Aufschluss mit Eagl, das an der Verbindung des CMV-Promoters und des Transposasegens und *Mlu*I (unterstrichen) spaltet, und durch Klonen in die entsprechenden Orte von pCVM/SB erzeugt. Ein Teil der Mutationssequenz mit den kodierten Aminosäuren ist folgender:

54 55 56 57 58 59 60 61 62
G CCG TCA TAC CGC CCA GGA AGG AGA CGC GT
P S Y R P G R R R

Die Mutation ist eine einzige Aminosäurenveränderung in Position 58, die jetzt ein Prolin (fett gedruckt) anstelle des ursprünglichen Serins ist.

Alle Konstrukte, die die Mutationen aufweisen, werden auf die richtige Expression durch Hybridisierungen (Western hybridizations) hin geprüft, wobei ein polyklonaler Anti-SB-Antikörper verwendet wird. Alle drei obigen Mutationstransposasen vermitteln die Transposition unter Verwendung des pT/Neo-Spenderkonstrukts (Ivics et al., 1997) in menschlichen HeLa-Zellen bei Stärken, die mit Wildtyp-SB-Transposase vergleichbar sind. Die vergleichbaren Niveaus liegen hier im Bereich von 90 % bis 110 % der Aktivität von Wildtyp-SB-Transposase. Insgesamt zeigen diese Daten, dass bestimmte Veränderungen am Transposasepeptid vorgenommen werden können, ohne dass seine funktionellen Eigenschaften negativ beeinflusst werden.

Zusammengefasst besitzt das SB-System mehrere Vorteile für den Gentransfer bei Wirbeltieren:
- SB kann unterschiedlichste Wirbeltierzellen umwandeln;
- weil SB ein Transposon auf DNA-Basis ist, besteht keine Notwendigkeit der reversen Transkription des Transgens, die Mutationen in retrovirale Vektorstämme einbringt;
- SB ist offensichtlich nicht in seiner Fähigkeit beschränkt, DNA jeder Sequenz zu transponieren;
- SB-Vektoren weisen keine strikten Größenbeschränkungen auf;
- da Transposonen nicht infektiös sind, sind Vektoren auf Transposonbasis nicht replikationskompetent und breiten sich deshalb nicht auf andere Zellpopulationen aus;
- SB erfordert lediglich etwa 230 bp transposoninvertierte Repetitions-DNA, die zur Mobilisierung ein Transgen auf jeder Seite flankiert;
- SB-Vektoren sind in der Transkription neutral und verändern somit nicht die endogene Genfunktion;
- Transposition ist induzierbar und erfordert nur das Transposaseprotein; auf diese Weise können Position und Moment des Sprungs durch Kontrolle der Transposaseexpression einfach gesteuert werden.
- es wird erwartet, dass SB in der Lage ist, nicht teilende Zellen zu transduzieren, weil die Transposase ein Kernlokalisierungssignal enthält, durch das Transposon/Transposasekomplexe aktiv in Zellkerne transportiert werden können;
- SB bewirkt die stabile Integration von Genen in Einzelkopie in Chromosomen; damit wird die Grundlage langfristiger Expression durch mehrere Generationen transgener Zellen und Organismen gelegt;
- sobald sie integriert sind, wird erwartet, dass SB-Elemente sich als stabile, dominante genetische Determinanten in den Genomen umgewandelter Zellen verhalten, weil 1) die Präsenz von SB-Transposase in Zellen nur vorübergehend und auf ein Zeitfenster beschränkt ist, wenn die Transposition katalysiert wird, und 2) keine endogene Transposasequelle in Wirbeltierzellen nachgewiesen wird, die integrierte SB-Elemente aktivieren und mobilisieren könnte;
- mit Ausnahme einiger Fischarten gibt es in Wirbeltiergenomen keine endogenen Sequenzen mit ausreichender Homologie zu SB, die die Rekombination und Freisetzung transpositional kompetenter (autonomer) Elemente ermöglichen würde;
- zur effizienten Einbringung in Zellen könnte SB mit DNA-Zufuhragenzien wie Adenoviren und Liposomen kombiniert werden;
- da SB ein Vektor auf Plasmidbasis ist, ist seine Produktion einfach, preiswert und kann vergrößert werden.

### LITERATURHINWEISE

Ivics, Z., Hackett, P.B., Plasterk, R.H. & Izsvak, Z. Molecular reconstruction of Sleeping Beauty, a Tcl-like transposon from fish, and its transposition in human cells. *Cell* **91**, 501-510 (1997)
Izsvak, Z., Ivics, Z. & Plasterk, R.H., (2000) Sleeping Beauty, a wide horst-range transposon vector for genetic transformation in vertebrates. *J. Mol.Biol.* **302**, 93-102.
Luo, G., Ivics, Z. & Bradly, A. (1998). Chromosomal transposition of a Tcl/mariner-like element in mouse emobryonic stem cells. *Proc Natl Acad Sci U S A* **95**, 10769-10773.
Miller, A.D. (1997). Development and applications of retroviral vectors. In Retroviruses (Herausgeber Coffin, J.M., Hughes, S.H. & Vermus, H.E.) 843 pp. (Cold Spring Harbor Laboratory Press, New York).
Plasterk, R. H., Izsvák, Z. & Ivics, Z. (1999). Resident aliens: the Tcl/mariner superfamily of transposable elements. *Trend Genet.* 15, 326-32.
Yant, S.R., Meuse, L., Chiu, W., Ivics, Z., Izsvak, Z. & Kay, M.A. (2000) Somatic integration and long-term transgene expression in normal and haemophilic mice using a DNA transposon system. *Nat. Genent.* **25**, 35-41.

### BILDLEGENDEN

**Abb. 1. Abhängigkeit der Transposition von der Transposongröße.** Wirksamkeit der Transposition von SB-Elementen, die Insertionen unterschiedlicher Größe enthalten. Die Transposasequelle und Transpositionsanalyse entsprechen den Angaben in Bild 2, und die Transpositionseffizienz von T/neo, eines Transposons von 2,2 kb, wird auf 100 % eingestellt.
**Abb. 2. Darstellung eines "Sandwich"-SB-Transposonvektors und seine Transposition in menschliche HeLa-Zellen (A).** Vergleich des Wildtyp- (Konstrukt A) und des Sandwich- (Konstrukt B) Transposonvektors, die das gleiche Markergen aufweisen. Die internen Transposasebindungserzeuger im Sandwichelement sind zur Spaltung nicht in der Lage, so dass die individuellen Transposoneinheiten nicht in der Lage sind, selbst zu transponieren. Nur das vollständige, zusammengesetzte Element kann transponieren **(B)** Vergleich der entsprechenden Transpositionsfähigkeit der Kontrukte A und B in den menschlichen HeLa-Zellen. β bedeutet β-Galactosidase, ein Kontrollpeptid, SB ist "Sleeping Beauty"(Dornröschen)-Transposase. die Zahlen sind G-418-resistente Zellkolonien pro 10⁶ transfizierter Zellen.
**Abb. 3. Genauigkeit der Transposition von Sleeping Beauty in menschlichen HeLa-Zellen. (A)** Genetische Analyse auf Positiv-Negativ-Selektion für SB-Transposition in kultivierten Zellen. Die IR/DR-Sequenzen, die die SB-Transposonen flankieren, sind angegeben. (**B**) Zahlen der pro 2x10⁶ transfizierte Zellen erzielten Zellklone unter G-418 gegenüber G-418 plus Gancyclovir-Selektion in Abwesenheit und Anwesenheit von SB-Transposase.
**Abb. 4. Die Häufigkeit der Transposition von** ***Sleeping Beauty*** **ist direkt proportional der Höhe der Transposaseexpression.** Helferplasmide wurden mit pT/neo in kultivierte HeLa-Zellen cotransfiziert, und die verschiedenen Promotoren, die zur Aktivierung der Expression von SB-Transposase verwendet wurden, sind angegeben. Die Zahlen der Transformanten in der oberen Graphik stellen die Zahlen der G-418-resistenten Zellkolonien pro Schale dar. Die Graphik unten stellt die CAT-Aktivitäten dar, die unter denselben Versuchsbedingungen mit denselben Promotoren erzielt werden wie sie für die Transpositionsanalyse verwendet werden.

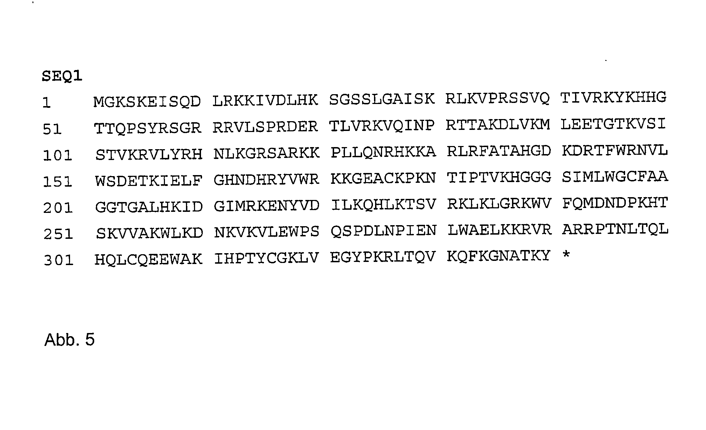

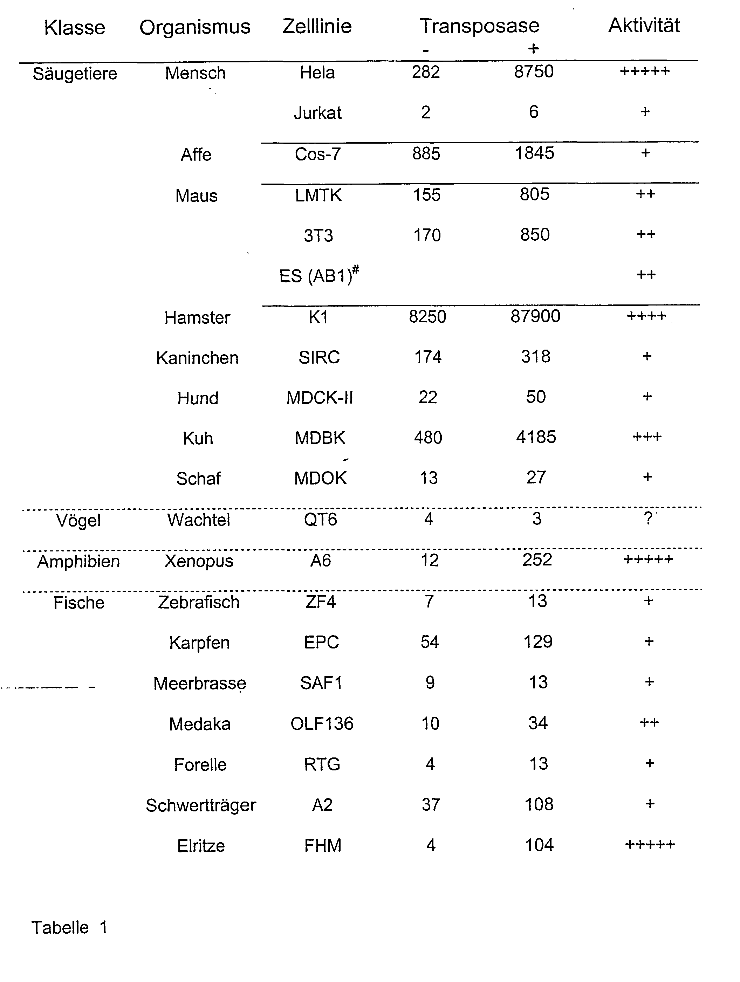

**Abb. 5**. Aminosäuresequenz des *Sleeping beauty* Transposons

**Tabelle 1.** ***Sleeping Beauty*** **ist bei diversen Wirbeltierarten wirksam.** Die Zahlen der Zellklone pro 5 x 10⁶ transfizierter Zellen, die in verschiedenen Wirbeltier-Zellstämmen unter G-418-Selektion in Abwesenheit und Gegenwart von SB-Transposase erzielt werden, sind aufgezeigt. Die Zellen wurden mit dem pCMV-SB-Transposase-Epressionshelferplasmid und dem pT/neo-Spenderkonstrukt⁹ cotransfiziert. Die Transpositionseffizienz wird als das Verhältnis zwischen der Zahl der G-418-resistenten Zellklone, die in Gegenwart von SB-Transposase erzielt werden, gegenüber der Zahl der in Abwesenheit von SB-Transposase erzielten Zellklone ausgedrückt. + 1-3fach, ++ 3-5fach, +++ 5-10fach, ++++ 10-20-fach, +++++ > 20fach. Die dargestellten Zahlen sind Mittelwerte, die Abweichung vom Mittel beträgt 10 %.

## Patentansprüche

1. Transposonvektor zur stabilen Insertion von DNA in Chromosomen lebender Wirbeltiere, **dadurch gekennzeichnet, dass** die zur transferierende DNA
- von zwei kompletten Sleeping Beauty-Elementen entweder in gerader oder umgekehrter Richtung zueinander flankiert wird und
- ein Sleeping Beauty-Element durch zwei IR/DR-Sequenzen in umgekehrter Richtung zueinander definiert wird, wobei
- jedes Element zumindest zwei Transposasebindungsorte enthält.

2. Transposonvektor nach Anspruch 1, **dadurch gekennzeichnet, dass** die Transposase zumindest eine 90 %-ige, bevorzugt eine 95%-ige, besonders bevorzugt eine 98%-ige Sequenzidentität mit SEQ ID NO:1 hat.

3. Transposonvektor nach Anspruch 1, wobei die zu transferierende DNA vom Fisch, vom Frosch, vom Reptil, vom Vogel, von Säugetieren oder vom Menschen stammt.

4. Transposonvektor nach Anspruch 1, wobei die Transposasequelle ein Transposaseprotein, eine mRNA oder eine Plasmid-DNA ist.

5. Transposonvektor nach Anspruch 1, wobei der Gentransfer mit Liposomen, mit PEI (Polyethylenimin), mit Adenovirus-Polylysin-DNA-Komplexen - rezeptorvermitteltem Gentransfer, mit einem rekombinanten Retrovirus, wie mit einem rekombinanten Adenovirus, mit einem rekombinanten Herpesvirus oder mit einem rekombinanten adenoassoziierten Virus kombiniert werden kann.

6. Transposonvektor nach Anspruch 1, wobei die zu transferierende DNA zur Korrektur eines einzelnen Gendefekts verwendet werden kann, oder die zu transferierende DNA ein therapeutisches Gen für die Krebsgentherapie ist.

7. Verwendung eines Transposonvektors nach Anspruch 1 zur Herstellung eines Mittels zu Diagnosezwecken.

8. Verwendung eines Transposonvektors nach Anspruch 1 zur Herstellung eines Arzneimittels zur Gentherapie.

## Revendications

1. Vecteur transposon pour l'insertion stable d'ADN dans des chromosomes d'animaux vertébrés vivants, **caractérisé en ce que** l'ADN à transférer
- est flanqué de deux éléments Sleeping Beauty complets, soit avec la même orientation, soit avec des orientations opposées, et
- un élément Sleeping Beauty est défini par deux séquences IR/DR avec des orientations opposées, où
- chaque élément contient au moins deux sites de liaison à la transposase.

2. Vecteur transposon selon la revendication 1, **caractérisé en ce que** la transposase a au moins une identité de séquence de 90 %, de préférence de 95 %, de façon particulièrement préférée de 98 % avec SEQ ID NO. 1.

3. Vecteur transposon selon la revendication 1, où l'ADN à transférer provient d'un poisson, d'une grenouille, d'un reptile, d'un oiseau, de mammifères ou de l'homme.

4. Vecteur transposon selon la revendication 1, où la source de transposase est une protéine transposase, un ARNm ou un ADN plasmidique.

5. Vecteur transposon selon la revendication 1, où le transfert de gène peut être combiné avec des liposomes, avec une PEI (polyéthylène-imine), avec un transfert de gène par l'intermédiaire d'un récepteur -complexes adénovirus-polylysine-ADN, avec un rétrovirus recombinant, comme avec un adénovirus recombinant, avec un herpèsvirus recombinant ou avec un virus adéno-associé recombinant.

6. Vecteur transposon selon la revendication 1, où l'ADN à transférer peut être utilisé pour corriger un défaut génétique particulier, ou l'ADN à transférer est un gène thérapeutique pour la thérapie génique contre le cancer.

7. Utilisation d'un vecteur transposon selon la revendication 1 pour la préparation d'un agent destiné à des applications diagnostiques.

8. Utilisation d'un vecteur transposon selon la revendication 1 pour la préparation d'un médicament destiné à la thérapie génique.

## Claims

1. Transposon vector for the stable insertion of DNA in chromosomes of living vertebrates, **characterised in that** the DNA to be transferred
- is flanked by two complete Sleeping Beauty elements either direct or inverted and
- a Sleeping Beauty element is defined by two inverted IR/DR sequences in which
- each element contains at least two transposase binding sites.

2. Transposon vector as claimed in claim 1, **characterised in that** the transposase sequence is at least 90%, preferably 95%, even more preferably 98% identical to SEQ ID No:1.

3. Transposon vector as claimed in claim 1, in which the DNA to be transferred originates from fish, frogs, reptiles, birds, mammals or humans.

4. Transposon vector as claimed in claim 1, in which the transposase source is a transposase protein, a mRNA or a plasmid DNA.

5. Transposon vector as claimed in claim1, whereby the gene transfer can be combined with liposomes, PEI (polyethlyene imine), adenovirus polylysine DNA complexes - receptor induced gene transfer, a recombinant retrovirus, as well as with a recombinant adenovirus, a recombinant herpes virus or with a recombinant adeno-associated virus.

6. Transposon vector as claimed in claim 1, in which the DNA to be transferred can be used to correct an individual gene defect or the DNA to be transferred is a therapeutic gene for cancer gene therapy.

7. Use of a transposon vector as claimed in claim 1 to produce a means for diagnostic purposes.

8. Use of a transposon vector as claimed in claim 1 to produce a medicament for gene therapy.
